# FASCICULE DE BREVET EUROPEEN

(11) **EP 2 358 662 B1**
(45) Date de publication et mention de la délivrance du brevet: **30.07.2014**
(21) Numéro de dépôt: 09802175.1
(22) Date de dépôt: 15.12.2009
(51) Int. Cl.: C07C 227/08, C07C 229/08

(54) **PROCEDE D'AMMONOLYSE DE L'ACIDE 11-BROMOUNDECANOÏQUE**
VERFAHREN ZUR AMMONOLYSE VON 11-BROMUNDECANSÄURE
PROCESS FOR THE AMMONOLYSIS OF 11-BROMOUNDECANOIC ACID

(30) Priorité: 19.12.2008 FR 0807259
(43) Date de publication de la demande: 24.08.2011
(73) Titulaire: Arkema France, 92700 Colombes (FR)
(72) Inventeur: PEES, Bernard, F-13190 Allauch (FR); LEBRUN, Stephanie, F-27550 Nassandres (FR)
(74) Mandataire: Lhoste, Catherine
(86) Numéro de dépôt international: PCT/FR2009/052536
(87) Numéro de publication internationale: WO 2010/070228

(56) Documents cités:
- GB-A- 591 027
- GUDADHE, SHRIKANT B. ET AL: "Kinetic studies of amination of 11-bromoundecanoic acid" INDUSTRIAL & ENGINEERING CHEMISTRY PROCESS DESIGN AND DEVELOPMENT, 25(2), 354 -7 CODEN: IEPDAW; ISSN: 0196-4305, 1986, XP002531389 cité dans la demande

## Description

La présente invention concerne un procédé d'ammonolyse de l'acide 11-bromoundécanoïque, effectué dans des conditions permettant de limiter les réactions secondaires à l'origine des impuretés, notamment de type amines secondaires, et en réduisant considérablement le temps de réaction.

A partir de l'huile de ricin on peut isoler un acide halogéné, l'acide 11-aminoundécanoïque, notamment par l'intermédiaire d'une réaction effectuée en présence d'ammoniac, appelée ammonolyse. L'acide 11-aminoundécanoïque ainsi obtenu est utilisé notamment comme monomère dans la synthèse, par condensation, de polyamides homopolymères comme le Rilsan^{®} ou PA11. Rilsan® PA 11 est le seul polymère à fournir des performances haut de gamme dans différents domaines physiques majeurs, parmi lesquelles la résistance chimique et aux hydrocarbures, la résistance aux chocs, à l'éclatement, à l'abrasion et aux fissures, la flexibilité, une température de service élevée et un vieillissement à long terme. Les propriétés de ces polymères sont modifiées lorsque le taux d'impuretés présentes dans le milieu de réaction lors de la condensation dépasse un certain seuil. Il est donc souhaitable de limiter au maximum la formation de produits secondaires.

Il est connu de faire réagir un acide halogéné avec une solution d'ammoniac pour obtenir l'acide amino-carboxylique correspondant, suivant la méthode d'Hoffmann pour la préparation des amines. Le document FR 988 699 décrit un procédé pour la préparation d'acides amino-carboxyliques en soumettant un acide halogéné à l'action de solutions aqueuses, alcooliques ou hydro-alcooliques d'ammoniaque. La réaction a lieu à des températures relativement basses. Sa vitesse croît avec la température. En revanche, le rendement en amino-acides diminue avec l'élévation de la température. L'exemple de réalisation cité dans ce document décrit un procédé de préparation de l'acide 10-aminodécanoïque consistant à introduire de l'acide 10-bromoundécanoïque à l'état fondu dans une solution aqueuse d'ammoniaque à 25% en poids de ce dernier, à agiter le mélange et à le maintenir à environ 15°C. La réaction est complète après environ 6 jours et présente un rendement de 77%.

Le document GB 591027 décrit un procédé de fabrication d'acide 11-aminoundécanoïque à partir de l'acide 11-bromoundécanoïque, en dispersant par agitation de l'acide 11-bromoundécanoïque dans une solution aqueuse d'ammoniac à 25% en poids, et en maintenant le mélange ainsi obtenu à une température constante, de 30°C dans l'exemple 1 de ce document. La réaction est complète après 100 à 120 heures et présente un rendement de 75%. Dans l'exemple 2 de ce même document, le mélange est maintenu à une température constante de 60°C, la réaction est complète après 10 heures mais présente un rendement de 53%.

Une hausse de la température de réaction entraîne par ailleurs une augmentation non négligeable de la quantité d'impuretés formées, ce qui est préjudiciable pour les propriétés du polyamide synthétisé à partir de cet effluent. Le fait de soumettre cet effluent à des traitements ultérieurs de purification et séparation de l'amino-acide induit une augmentation non négligeable du coût de fabrication des polyamides.

Gudadhe et al. (Ind. Eng. Chem. Process Des. Dev. 1986, 25, 354-357) ont étudié la cinétique de la réaction d'amination de l'acide 11-bromoundécanoïque. Les résultats figurant dans le tableau I de ce document montrent qu'une augmentation de la température de réaction de 30 à 50°C conduit à une augmentation de la vitesse de réaction, accompagnée d'une diminution du rendement en l'acide 11-aminoundécanoïque. Ceci serait dû à l'augmentation de la quantité d'impuretés formées en parallèle.

Lors de la réaction d'ammonolyse de l'acide 11-bromoundécanoïque, il est possible de limiter très fortement les réactions parallèles à l'origine des principales impuretés (acide 11-hydroxyundécanoique HO-(CH₂)₁₀-COOH et acide amino-diundécanoique NH[(CH₂)₁₀-COOH]₂), en réduisant la concentration des acides 11-bromoundécanoïque et 11-aminoundécanoïque dans le milieu. Pour cela, la réaction d'ammonolyse est réalisée en milieu aqueux et hétérogène. Ce faisant, l'acide 11-bromoundécanoïque se solubilise dans la phase aqueuse sous la forme de sel d'ammonium dont la solubilité à basse température (inférieure à 30°C) est très faible. Il est possible de restreindre avantageusement la réaction d'aminolyse de l'acide 11-bromoundécanoïque par l'acide 11-aminoundécanoïque - conduisant à l'acide amino-diundécanoique - en utilisant un très large excès d'ammoniaque. La très forte proportion d'ammoniaque dans le milieu comparativement à l'acide 11-aminoundécanoïque réduit considérablement la probabilité de rencontre entre ce dernier et l'acide 11-bromoundécanoïque, limitant très significativement leurs réactivités mutuelles.

Cependant, la conduite de la réaction à basse température, si elle permet de réduire les réactions secondaires et d'obtenir sélectivement l'acide 11-aminoundécanoique, entraîne également des durées de réaction (avec consommation totale de l'acide 11-bromoundécanoïque) extrêmement longues, incompatibles avec la mise en oeuvre à l'échelle industrielle de cette réaction (95h pour une réaction isotherme à 22°C).

La présente invention se propose de pallier les inconvénients présentés par les procédés d'ammonolyse de l'acide 11-bromoundécanoïque connus.

L'objet de la présente invention est de fournir un procédé d'ammonolyse de l'acide 11-bromoundécanoïque qui permette d'obtenir de l'acide 11-aminoundecanoïque avec une production de sous-produits considérablement limitée et pendant un temps de réaction plus court compatible avec la conduite industrielle de la réaction.

Il a maintenant été trouvé que seule la température initiale de la réaction d'ammonolyse de l'acide 11-bromoundécanoïque gouverne la formation des espèces secondaires. Il n'est donc pas nécessaire de conduire la réaction selon une isotherme à basse température, mais de soumettre le milieu à une montée en température régulière tout en observant un palier initial à basse température (15 à 25°C).

L'invention a pour objet un procédé de fabrication de l"acide 11-aminoundécanoïque à partir de l'acide 11-bromoundécanoïque, comprenant les étapes suivantes:
i) une étape de dispersion de l'acide 11-bromoundécanoïque, fondu ou non, dans une solution aqueuse d'ammoniac, et
ii) une étape d'ammonolyse par réaction de 11-bromoundécanoïque avec l'ammoniaque en excès, dans des conditions d'agitation du milieu réactionnel et de chauffage progressif de celui-ci, suffisantes pour permettre l'obtention de l'acide 11-aminoundécanoïque avec une consommation totale de l'acide 11-bromoundécanoïque en moins de 80h tout en limitant la formation d'acide amino-diundécanoique.

Le procédé faisant l'objet de la présente invention, avec une réaction conduite à température croissante et non isotherme, présente l'avantage d'atteindre une conversion très élevée en acide 11-aminoundécanoïque, tout en limitant la quantité d'impuretés formées (principalement de l'acide amino-diundécanoïque, dont le taux dans le brut réactionnel est inférieur à 3500 ppm, de préférence inférieur à 2500 ppm), et en réduisant considérablement le temps de réaction.

D'autres caractéristiques et avantages ressortiront de la description détaillée du procédé d'ammonolyse de l'acide 11-bromoundécanoïque selon l'invention qui va suivre et des figures annexées dans lesquelles :
- la figure 1 illustre une variante de réalisation du procédé d'ammonolyse selon l'invention, dans laquelle l'étape ii) se déroule en continu dans une batterie de réacteurs montés en série R1, R2, ... Rn, 2 ≤ n ≤ 25, chacun de ces réacteurs étant maintenu indépendamment à une température constante et contrôlée T1, T2, ... Tn respectivement,
- la figure 2 illustre une autre variante de réalisation du procédé d'ammonolyse selon l'invention, dans laquelle l'étape ii) se déroule en continu dans une batterie de réacteurs montés en parallèle R'1, R'2, ... R'n, 2 ≤ n ≤ 25, chacun de ces réacteurs étant soumis à une température variable,
- la figure 3 illustre une troisième variante de réalisation du procédé d'ammonolyse selon l'invention, dans laquelle l'étape ii) se déroule en discontinu (mode batch) dans un réacteur soumis à une température variable Tf,
- la figure 4 illustre la variation de la température en fonction du temps, ainsi que la cinétique de la réaction (variation de l'avancement réactionnel en fonction du temps) dans un essai en batch selon l'invention, avec montée en température en six paliers,
- la figure 5 illustre la variation de la température en fonction du temps, ainsi que la cinétique de la réaction (variation de l'avancement réactionnel en fonction du temps) dans un essai en batch comparatif, avec conduite isotherme de la réaction à 22°C,
- la figure 6 illustre la variation de la température en fonction du temps, ainsi que la cinétique de la réaction (variation de l'avancement réactionnel en fonction du temps) dans un essai en batch comparatif, avec conduite isotherme de la réaction à 32°C.

La présente invention a pour objet un procédé d'ammonolyse de l'acide 11-bromoundécanoïque comprenant les étapes suivantes:
i) une étape de dispersion de l'acide 11-bromoundécanoïque, fondu ou non, dans une solution aqueuse d'ammoniac, l'ammoniaque étant en excès et
ii) une étape d'ammonolyse par réaction de 11-bromoundécanoïque avec l'ammoniaque, dans des conditions d'agitation du milieu réactionnel et de chauffage progressif de celui-ci, suffisantes pour permettre l'obtention de l'acide 11-aminoundécanoïque avec une consommation totale de l'acide 11-bromoundécanoïque en moins de 80h.

L'ammonolyse de l'acide 11-bromoundécanoïque s'effectue suivant la suite de réactions suivante :
- formation et solubilisation du sel d'ammonium de l'acide 11-bromoundécanoïque :
   Br-(CH₂)₁₀-COOHₛ + NH_{3aq} → Br-(CH₂)₁₀-COO⁻, NH₄⁺ₛ → Br-(CH₂)₁₀-COO⁻, NH₄⁺_{aq}
- ammonolyse du sel d'ammonium de l'acide 11-bromoundécanoïque :
   Br-(CH₂)₁₀-COO⁻, NH₄⁺_{aq} + NH_{3aq} → NH₂-(CH₂)₁₀-COO⁻, NH₄⁺_{aq}
- hydrolyse du sel d'ammonium de l'acide 11-aminoundécanoïque et précipitation de l'acide 11-aminoundécanoïque :
   NH₂-(CH₂)₁₀-COO⁻, NH₄⁺_{aq} ⁺ H₂O → NH₂-(CH₂)₁₀-COOH_{aq} → NH₂-(CH₂)₁₀-COOHₛ
- hydroxylation du sel d'ammonium de l'acide 11-bromoundécanoïque :
   Br-(CH₂)₁₀-COO⁻, NH₄⁺_{aq} + OH⁻_{aq} →HO-(CH₂)₁₀-COO⁻, NH₄⁺_{aq}
- aminolyse du sel d'ammonium de l'acide 11-aminoundécanoïque :
   Br-(CH₂)₁₀-COO⁻, NH₄⁺_{aq} + NH₂-(CH₂)₁₀-COC)⁻, NH₄⁺_{aq} → NH[(CH₂)₁₀-COO⁻]₂, 2 NH₄⁺_{aq}

L'étape i) se déroule par dispersion de l'acide 11-bromoundécanoïque (désigné ci-après par l'abréviation Br11) fondu, préférentiellement à une température de 60°C à 100°C, dans l'ammoniaque concentré (préférentiellement de 20 à 50% en poids), en excès par rapport au Br11 (soit un ratio molaire Br11:NH₃ compris entre 1:10 et 1:60, préférentiellement de 1:30) à une température allant de 0°C à 10°C, via un système d'injection.

De manière caractéristique, la réaction d'ammonolyse de l'acide 11-bromoundécanoïque a lieu dans des conditions spécifiques de température, à savoir une conduite de la réaction à température croissante et non isotherme. Plus spécifiquement, le milieu réactionnel est progressivement chauffé (soit grâce à un programme de températures croissantes, soit par déversement du milieu réactionnel dans une suite de réacteurs maintenus isolément à des températures fixes mais croissantes entre deux réacteurs consécutifs). On peut ainsi accélérer la cinétique réactionnelle et réduire le temps de réaction, à consommation totale de l'acide 11-bromoundécanoïque, de façon très significative, à savoir à moins de 80h, et de préférence à moins de 75h.

Avantageusement, le procédé selon l'invention permet de réduire la quantité de produits non désirés formés en parallèle, notamment la quantité d'acide amino-diundécanoïque, dont le taux dans le brut réactionnel obtenu reste inférieur à 3500 ppm, de préférence inférieur à 2500 ppm.

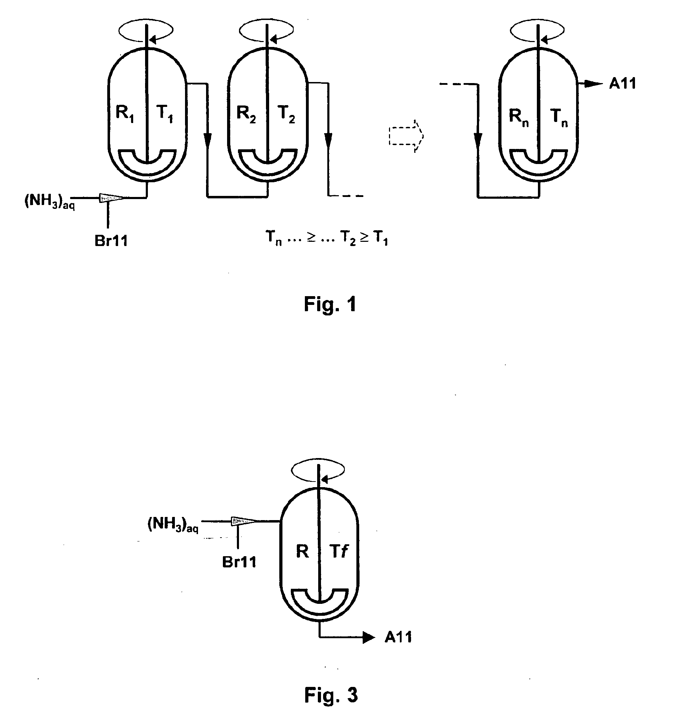

Selon une première variante de réalisation, l'étape ii) se déroule avantageusement en continu dans une batterie de réacteurs en série R1, R2, ... Rn, 2 ≤ n ≤ 25, maintenus indépendamment à une température contrôlée (T1, T2, ... Tn) - comme montré dans la figure 1 annexée. Le profil de température de la batterie de réacteurs se caractérise par une température croissante entre réacteurs consécutifs permettant de travailler entre 15 et 25°C pour le premier réacteur (celui recevant la dispersion d'acide 11-bromoundécanoïque dans l'ammoniaque) et une température de 26 à 40°C pour le réacteur final. Dans cette variante de réalisation, le milieu réactionnel est transféré d'un réacteur donné au réacteur consécutif et la durée de séjour moyenne dans chaque réacteur étant de 1 h à 30 h, la durée totale de la réaction permettant la consommation totale d'acide 11-bromoundécanoïque de 20 à 80h.

Le contrôle de la température de chaque réacteur se fait préférentiellement en combinant le contrôle de la température d'entrée des ingrédients (acide 11-bromoundécanoïque et ammoniaque) lors de l'étape i), la maîtrise du dégazage de l'ammoniac excédentaire dans chaque réacteur de l'étape ii) (phénomène endothermique permettant de réfrigérer le milieu) et l'utilisation de systèmes de chauffage thermorégulés des réacteurs de l'étape ii).

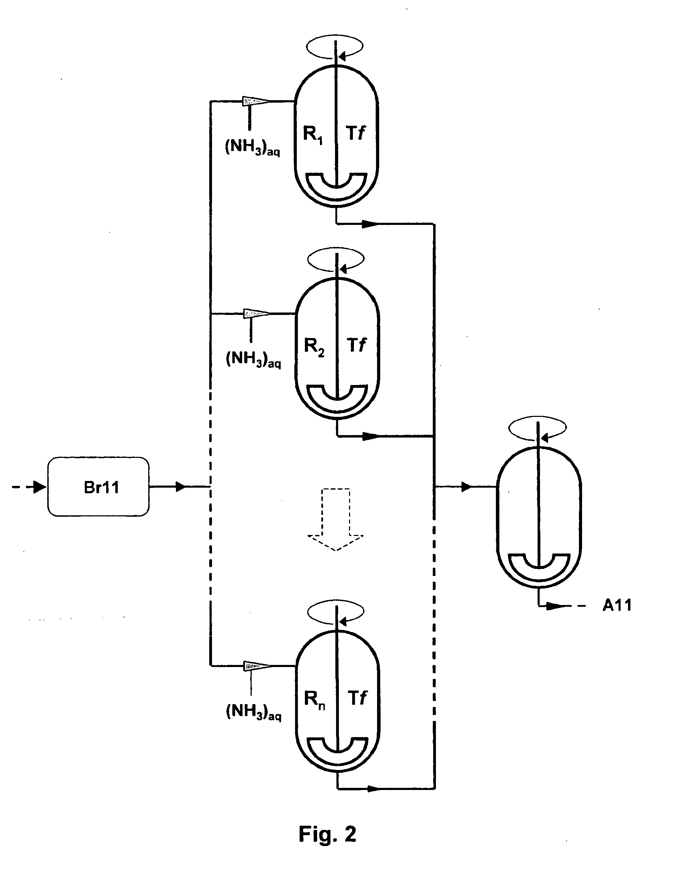

Selon une deuxième variante de réalisation, l'étape ii) se déroule dans une batterie de réacteurs en parallèle R1, R2, ... Rn, 2 ≤ n ≤ 25, maintenus indépendamment à une température variable - comme montré dans la figure 2 annexée. Chaque réacteur dispose d'un programme de montée en température sur une durée donnée dépendante des températures choisies. Dans chaque réacteur, la température initiale du programme est comprise entre 15 et 25°C et la température finale de 26 à 40°C. La durée de la réaction, permettant la consommation totale d'acide 11-bromoundécanoïque, varie de 20h à 80h selon les températures initiale et finale et le programme de montée en température. Afin de maintenir une alimentation et un soutirage constant, régulier et continu en entrée et en sortie de la batterie de réacteurs, chaque réacteur fonctionne selon des cycles chargement/réaction/vidange avec un décalage de temps équivalent à la durée réactionnelle divisée par le nombre de réacteurs (n).

Selon une troisième variante de réalisation, l'étape ii) se déroule en discontinu (mode batch) dans un réacteur maintenu à en température variable Tf- comme illustré à la figure 3 annexée. Ce réacteur dispose d'un programme de montée en température, sur un nombre donné de paliers ou une rampe de montée en température, sur une durée donnée dépendante des températures choisies. La température initiale du réacteur est comprise entre 15 et 25°C et la température finale du réacteur de 26 à 40°C. La durée de la réaction, permettant la consommation totale d'acide 11-bromoundécanoïque, varie de 20h à 80h selon les températures initiale et finale et le programme de montée en température.

Pour chacune des variantes de réalisation du procédé d'ammonolyse, il y a lieu de faire une distinction entre le temps de séjour et la durée de réaction : le temps de séjours dépend de la configuration de l'installation, caractérisée par exemple par le volume et le nombre de réacteurs, le sens d'écoulement, l'agitation, les débits, alors que le temps de réaction ne dépend que des paramètres propres à influencer la cinétique réactionnelle dont les températures et les concentrations.

Dans sa troisième variante, le procédé d'ammonolyse selon l'invention comprend également une étape iii) de filtration, lavage et d'essorage, effectuée sur l'acide 11-aminoundécanoïque obtenu à la fin de l'étape ii). Le produit de réaction ainsi obtenu est soumis à une étape iv) supplémentaire de purification, effectuée de préférence par la succession suivante d'opérations : redissolution, filtration, cristallisation, filtration, lavage et essorage.

Selon un mode de réalisation préféré, le procédé selon l'invention comprend également une étape v) de récupération de l'acide 11-aminoundécanoïque résiduel dans les différents filtrats et eaux de lavage obtenues dans les étapes iii) et/ou iv), par dégazage de l'ammoniac, extraction liquide/liquide, cristallisation, filtration et lavage.

Le procédé d'ammonolyse selon l'invention comprend en outre une étape finale de séchage de l'acide 11-aminoundécanoïque obtenu dans l'étape iv), éventuellement additionné de celui obtenu à l'étape v).

L'acide 11-aminoundécanoïque ainsi obtenu contient une quantité d'impuretés modérée, principalement de l'acide amino-diundécanoïque. Son utilisation comme monomère dans une réaction de condensation permet d'obtenir un polyamide PA11 ou Rilsan^{®} de très bonne qualité.

L'invention sera mieux comprise à la lecture des exemples de réalisation suivants, non limitatifs de l'invention.

### Exemple 1 selon l'invention : essai en batch avec montée en température en 6 paliers.

Dans un réacteur double enveloppe d'un litre équipé d'un axe d'agitation muni de deux hélices à 5 pales tournant à une vitesse de 400 rpm, on place 660 g d'ammoniaque à 32% à 0°C. On ajoute par un goutte à goutte rapide, 110 g d'acide 11-bromoundécanoïque fondu à 90°C. La réaction se déroule sous pression atmosphérique. L'addition de l'acide bromé réalisée, la consigne de température du milieu est augmentée à 22°C puis on impose au milieu réactionnel une montée en température de 22°C à 32°C en respectant 6 paliers de 12h30 à 22°C, 24°C, 26°C, 28°C, 30°C et 32°C.

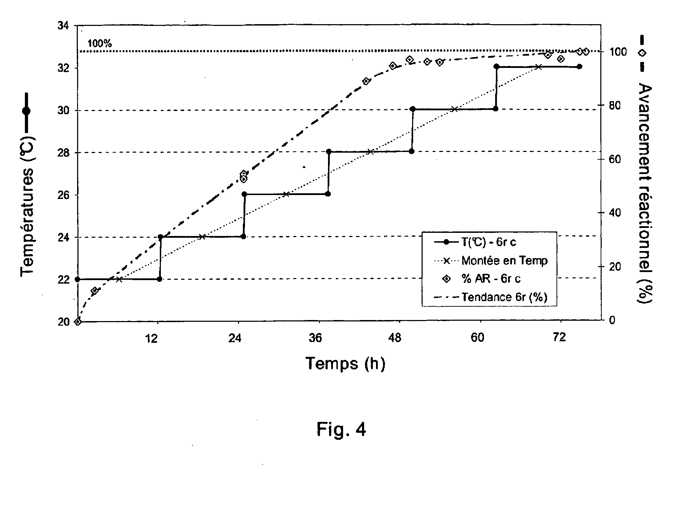

On définit l'avancement réactionnel (%AR) comme étant le taux d'acide 11-bromoundécanoïque consommé en fonction de la quantité d'acide initiale. On détermine la quantité d'acide bromé consommé par dosage des ions bromure libérés par la réaction dans le milieu par potentiométrie (titration à l'électrode d'argent et par nitrate d'argent). Le profil de température (température en fonction du temps) ainsi que la cinétique de la réaction (%AR en fonction du temps) sont présentés dans la figure 4.

Le brut réactionnel final est récupéré en l'état sous la forme d'une suspension coulante puis dégazé par chauffage. Le dosage de l'acide amino-diundécanoïque dans ce brut est réalisé par HPLC.

### Exemple 2 comparatif : essai en batch isotherme à 22°C

Dans un réacteur double enveloppe d'un litre équipé d'un axe d'agitation muni de deux hélices à 5 pales tournant à une vitesse de 400 rpm, on place 660 g d'ammoniaque à 32% à 0°C. On ajoute par un goutte à goutte rapide, 110 g d'acide 11-bromoundécanoïque fondu à 90°C. La réaction se déroule sous pression atmosphérique. L'addition de l'acide bromé réalisée, la consigne de température du milieu est augmentée à 22°C puis maintenu à 22°C jusqu'à consommation complète de l'acide 11-bromoundécanoïque.

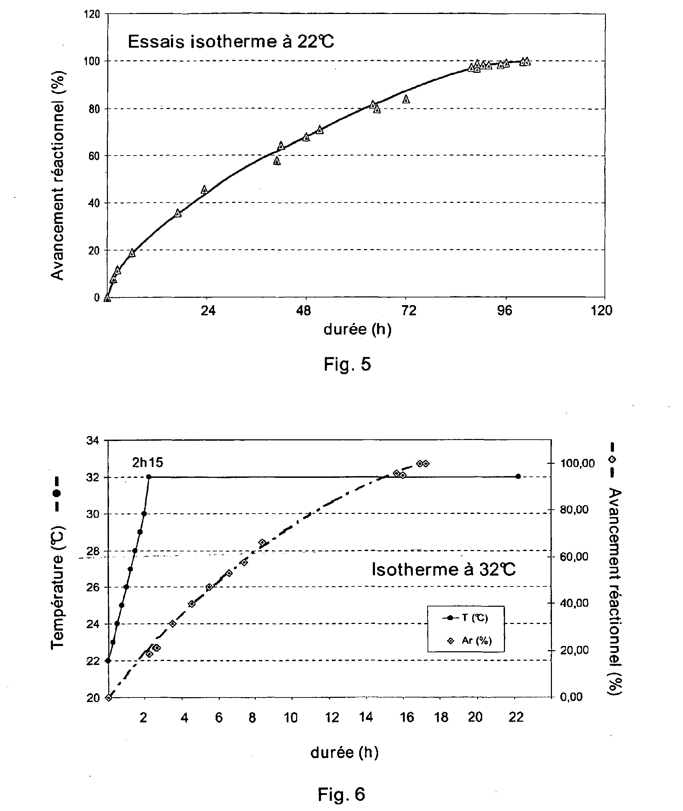

Le profil de température (température en fonction du temps) ainsi que la cinétique de la réaction (%AR en fonction du temps) sont présentés dans la figure 5.

Le brut réactionnel final est récupéré en l'état sous la forme d'une suspension coulante puis dégazé par chauffage. Le dosage de l'acide amino-diundécanoïque dans ce brut est réalisé par HPLC.

### Exemple 3 comparatif : essai en batch isotherme à 32°C

Dans un réacteur double enveloppe d'un litre équipé d'un axe d'agitation muni de deux hélices à 5 pales tournant à une vitesse de 400 rpm, on place 660 g d'ammoniaque à 32% à 0°C. On ajoute par un goutte à goutte rapide, 110 g d'acide 11-bromoundécanoïque fondu à 90°C. La réaction se déroule sous pression atmosphérique. L'addition de l'acide bromé réalisée, la consigne de température du milieu est augmentée progressivement à 32°C puis maintenue à 32°C jusqu'à consommation complète de l'acide bromé.

Le profil de température (température en fonction du temps) ainsi que la cinétique de la réaction (%AR en fonction du temps) sont présentés dans la figure 6.

Le brut réactionnel final est récupéré en l'état sous la forme d'une suspension coulante puis dégazé par chauffage. Le dosage de l'acide amino-diundécanoique dans ce brut est réalisé par HPLC.

Ces résultats sont présentés dans le tableau I suivant.

**Tableau I**

| | **Exemple 1** | **Exemple 2** | **Exemple 3** |
|---|---|---|---|
| | **6 paliers** | **iso 22°c** | **iso 32°C** |
| Durée (min) pour atteindre 100% de consommation Br11 | 4490 | 6060 | 1370 |
| A2 (mg/kg) | 2310 | 2051 | 3848 |

A2 représente le taux d'acide amino-diundécanoïque dans le brut réactionnel final, exprimé en ppm ou mg d'acide amino-diundécanoïque par kg de suspension.

Les exemples comparatifs 2 et 3 correspondent à deux cas pour lesquels la réaction d'ammonolyse se déroule à température constante jusqu'à consommation totale de l'acide 11-bromoundécanoique. Il faut ainsi maintenir le milieu réactionnel à 22°C durant 6060 min pour consommer totalement l'acide 11-bromo undécanoïque (exemple 2). Selon l'exemple 3, on peut réduire fortement la durée réactionnelle (-77,4%) en maintenant le milieu à une température plus élevée (32°C), mais la quantité d'acide amino-diundécanoïque formé s'en trouve très fortement augmentée (+87,6%).

L'exemple 1 selon l'invention correspond à une réaction d'ammonolyse réalisée à température croissante selon 6 paliers de température de 22°C à 32°C. Une comparaison avec l'exemple 2 montre qu'il est possible de réduire la durée de la réaction de (-25,9 %) en ne concédant qu'une légère augmentation d'acide amino-diundécanoïque de l'ordre de 12,6 %.

## Revendications

1. Procédé de fabrication d'acide 11-aminoundécanoïque à partir de l'acide 11-bromoundécanoïque, comprenant les étapes suivantes:
i) une étape de dispersion de l'acide 11-bromoundécanoïque, fondu ou non, dans une solution aqueuse d'ammoniac, et
ii) une étape d'ammonolyse par réaction de 11-bromoundécanoïque avec l'ammoniaque en excès, dans des conditions d'agitation du milieu réactionnel et de chauffage progressif de celui-ci, le milieu étant soumis à une montée en température régulière entre une température initiale de 15 à 25°C et une température finale de 26 à 40°C.

2. Procédé selon la revendication 1, dans lequel l'étape ii) se déroule en continu dans une batterie de réacteurs en série R1, R2, ... Rn, 2 ≤ n ≤ 25, R1 étant le réacteur qui reçoit la dispersion d'acide11-bromoundécanoïque dans l'ammoniaque, chacun de ces réacteurs étant maintenu indépendamment à une température constante et contrôlée T1, T2, ... Tn respectivement, la température de la batterie de réacteurs étant croissante entre deux réacteurs consécutifs.

3. Procédé selon la revendication 2 dans lequel la température T1 du réacteur R1 est de 15 à 25°C et la température Tn du réacteur final Rn est de 26 à 40°C.

4. Procédé selon l'une quelconque des revendications 2 et 3 dans lequel le contrôle de la température de chaque réacteur se fait en combinant le contrôle de la température d'entrée des ingrédients acide 11-bromoundécanoïque et ammoniaque lors de l'étape i), la maîtrise du dégazage de l'ammoniac excédentaire dans chaque réacteur de l'étape ii) et l'utilisation de systèmes de chauffage thermorégulés des réacteurs R1 à Rn.

5. Procédé selon l'une quelconque des revendications 2 à 4 dans lequel le milieu réactionnel étant transféré d'un réacteur donné au réacteur consécutif et la durée de séjour moyenne dans chaque réacteur étant de 1 h à 30 h, la durée totale de la réaction permettant la consommation totale d'acide 11-bromoundécanoïque de 20 à 80h.

6. Procédé selon la revendication 1, dans lequel l'étape ii) se déroule dans une batterie de réacteurs en parallèle R'1, R'2, ... R'n, 2 ≤ n ≤ 25, chacun de ces réacteurs étant soumis à une température variable.

7. Procédé selon la revendication 6 dans lequel la température initiale de chaque réacteur est de 15 à 25°C et la température finale de chaque réacteur est de 26 à 40°C.

8. Procédé selon l'une quelconque des revendications 6 et 7 dans lequel chaque réacteur fonctionne selon des cycles chargement/réaction/vidange avec un décalage de temps équivalent à la durée réactionnelle divisée par le nombre de réacteurs n, afin de maintenir une alimentation et un soutirage constant, régulier et continu en entrée et en sortie de la batterie de réacteurs.

9. Procédé selon la revendication 1, dans lequel l'étape ii) se déroule en discontinu dans un réacteur soumis à une température variable Tf, ledit réacteur disposant d'un programme en montée de température sur un nombre donné de paliers ou une rampe de montée en température dont la durée dépend de la température choisie.

10. Procédé selon la revendication 9 dans lequel la température initiale du réacteur est de 15 à 25°C et la température finale du est de 26 à 40°C.

11. Procédé selon l'une des revendications 6, 7, 9 et 10 dans lequel la durée de la réaction, permettant la consommation totale d'acide 11-bromoundécanoïque, varie de 20h à 80h selon les températures initiale et finale et le programme de montée en température.

12. Procédé selon l'une des revendications 9 à 11 dans lequel on impose au milieu réactionnel une montée en température de 22°C à 32°C en respectant 6 paliers de 12h30min à 22°C, 24°C, 26°C, 28°C, 30°C et 32°C.

13. Procédé selon l'une quelconque des revendications précédentes comprenant en outre une étape iii) de filtration, lavage et d'essorage, effectuée sur l'acide 11-aminoundécanoïque obtenu à la fin de l'étape ii).

14. Procédé selon la revendication 13 comprenant en outre une étape iv) de purification de l'acide 11-aminoundécanoïque obtenu à la fin de l'étape iii), de préférence par la succession suivante d'opérations : redissolution, filtration, cristallisation, filtration, lavage et essorage.

15. Procédé selon la revendication 14 comprenant en outre une étape v) de récupération de l'acide 11-aminoundécanoïque résiduel dans les différents filtrats et eaux de lavage obtenues dans les étapes iii) et/ou iv), par dégazage de l'ammoniac, extraction liquide/liquide, cristallisation, filtration et lavage.

16. Procédé selon l'une des revendications 14 et 15 comprenant en outre une étape finale de séchage de l'acide 11-aminoundécanoïque obtenu dans l'étape iv), éventuellement additionné de celui obtenu à l'étape v).

17. Procédé selon l'une quelconque des revendications 1 à 12, dans lequel le taux d'acide amino-diundécanoïque dans le brut réactionnel obtenu est inférieur à 3500 ppm, de préférence inférieur à 2500 ppm.

18. Procédé selon l'une quelconque des revendications 1 à 12 et 17, dans lequel la durée de l'étape d'ammonolyse conduisant à la consommation totale d'acide 11-bromoundécanoïque est inférieure à 75h.

## Patentansprüche

1. Verfahren zur Herstellung von 11-Aminoundecansäure aus 11-Bromundecansäure, das die folgenden Schritte umfasst :
i) einen Schritt des Dispergierens von geschmolzener oder nicht geschmolzener 11-Bromundecansäure in einer wässrigen Ammoniaklösung und
ii) einen Schritt der Ammonolyse durch Reaktion von 11-Bromundecansäure mit überschüssige wässrigem Ammoniak unter Rühren und allmählichem Erwärmen des Reaktionsmediums, wobei das Medium einer gleichmäßigen Temperatursteigerung zwischen einer Anfangstemperatur von 15 bis 25°C und einer Endtemperatur von 26 bis 40°C unterworfen wird.

2. Verfahren nach Anspruch 1, bei dem der Schritt ii) in einer Batterie von hintereinander angeordneten Reaktoren R1, R2, ... Rn, 2 ≤ n ≤ 25, kontinuierlich abläuft, wobei R1 der Reaktor ist, der die Dispersion von 11-Bromundecansäure in wässrigem Ammoniak aufnimmt, jeder dieser Reaktoren unabhängig bei einer konstanten und geregelten Temperatur T1, T2, ... Tn respektive gehalten wird und die Temperatur der Reaktorbatterie zwischen zwei aufeinanderfolgenden Reaktoren zunimmt.

3. Verfahren nach Anspruch 2, bei dem die Temperatur T1 des Reaktors R1 15 bis 25°C beträgt und die Temperatur Tn des letzten Reaktors Rn 26 bis 40°C beträgt.

4. Verfahren nach einem der Ansprüche 2 und 3, bei dem die Regelung der Temperatur jedes Reaktors durch Kombination der Regelung der Eintrittstemperatur der Bestandteile 11-Bromundecansäure und wässrigem Ammoniak in Stufe i), die Steurung der Entgasung des überschüssigen Ammoniaks in jedem Reaktor von Schritt ii) und die Verwendung von temperaturgeregelten Erwärmungssystemen der Reaktoren R1 bis Rn erfolgt.

5. Verfahren nach einem der Ansprüche 2 bis 4, bei dem bei Überführung des Reaktionsmediums von einem gegebenen Reaktor in den darauffolgenden Reaktor die mittlere Verweilzeit in jedem Reaktor 1 h bis 30 h beträgt und die Gesamtdauer der Reaktion, die den vollständigen Verbrauch von 11-Bromundecansäure erlaubt, 20 bis 80 h beträgt.

6. Verfahren nach Anspruch 1, bei dem der Schritt ii) in einer Batterie von parallel angeordneten Reaktoren R'1, R'2, ... R'n, 2 ≤ n ≤ 25, abläuft, wobei jeder dieser Reaktoren einer variablen Temperatur unterworfen wird.

7. Verfahren nach Anspruch 6, bei dem die Anfangstemperatur jedes Reaktors 15 bis 25°C beträgt und die Endtemperatur jedes Reaktors 26 bis 40°C beträgt.

8. Verfahren nach einem der Ansprüche 6 und 7, bei dem jeder Reaktor gemäß Beschickung/Reaktion/Entleerung-Zyklen mit einer Zeitversetzung die der Reaktionsdauer dividiert durch die Zahl der Reaktoren n entspricht, arbeitet, um einen konstanten, gleichmäßigen und kontinuierlichen Eintrag und Austrag am Eingang und am Ausgang der Reaktorbatterie aufrechtzuerhalten.

9. Verfahren nach Anspruch 1, bei dem Schritt ii) in einem einer variablen Temperatur Tf unterworfenen Reaktor diskontinuierlich abläuft, wobei der Reaktor über ein Programm zur Steigerung der Temperatur über eine gegebene Zahl von Stufen oder eine Rampe zur Steigerung der Temperatur, deren Dauer von der gewählten Temperatur abhängt, verfügt.

10. Verfahren nach Anspruch 9, bei dem die Anfangstemperatur des Reaktors 15 bis 25°C beträgt und die Endtemperatur des Reaktors 26 bis 40°C beträgt.

11. Verfahren nach einem der Ansprüche 6, 7, 9 und 10, bei dem die Dauer der Reaktion, die den vollständigen Verbrauch von 11-Bromundecansäure erlaubt, je nach der Anfangs- und Endtemperatur des Tenperatursteigerungsprogramms von 20 bis 80 h variiert.

12. Verfahren nach einem der Ansprüche 9 bis 11, bei dem man das Reaktionsmedium einer Temperatursteigerung von 22°C bis 32°C über 6 Stufen mit einer Dauer von 12 h 30 min bei 22° C, 24°C, 26°C, 28°C, 30°C und 32°C unterwirft.

13. Verfahren nach einem der vorhergehenden Ansprüche, das außerdem einen Schritt iii) des Filtrierens, Waschens und Trockenziehens umfasst, der an der am Ende von Schritt ii) erhaltenen 11-Aminoundecansäure durchgeführt wird.

14. Verfahren nach Anspruch 13, das außerdem einen Schritt iv) des Reinigens der am Ende von Schritt iii)) erhaltenen 11-Aminoundecansäure umfasst, vorzugsweise durch die folgende Abfolge von Arbeitsgängen: Wiederauflösen, Filtrieren, Kristallisieren, Filtrieren, Waschen und Trockenziehen.

15. Verfahren nach Anspruch 14, das außerdem einen Schritt v) des Gewinnens der in den verschiedenen in den Schritten iii) und/oder iv) erhaltenen Filtraten und Waschwässern verbliebenen 11-Aminoundecansäure durch Entgasen des Ammoniaks, Flüssig/Flüssig-Extraktion, Kristallisieren, Filtrieren und Waschen umfasst.

16. Verfahren nach einem der Ansprüche 14 und 15, das außerdem einen letzten Schritt des Trocknens der in Schritt iv) erhaltenen 11-Aminouridecansäure, gegebenenfalls unter Zugabe der in Schritt v) erhaltenen 11-Aminoundecansäure, umfasst.

17. Verfahren nach einem der Ansprüche 1 bis 12, bei dem der Aminodiundecansäuregehalt des erhaltenen rohen Reaktionsprodukts weniger als 3500 ppm und vorzugsweise weniger als 2500 ppm beträgt.

18. Verfahren nach einem der Ansprüche 1 bis 12 und 17, bei dem die Dauer des Ammonolyseschritts, die zum vollständigen Verbrauch von 11-Bromundecansäure führt, weniger als 75 h beträgt.

## Claims

1. Process for the manufacture of 11-aminoundecanoic acid from 11-bromoundecanoic acid, comprising the following stages:
i) a stage of dispersion of molten or non-molten 11-bromoundecanoic acid in an aqueous ammonia solution, and
ii) a stage of ammonolysis by reaction of 11-bromoundecanoic acid with excess aqueous ammonia under conditions of stirring the reaction medium and of gradual heating of the latter, the medium being subjected to a regular rise in temperature between a starting temperature of 15 to 25°C and a final temperature of 26 to 40°C.

2. Process according to Claim 1, in which stage ii) takes place continuously in an array of reactors in series R1, R2, ... Rn, 2 ≤ n ≤ 25, R1 being the reactor which receives the dispersion of 11-bromoundecanoic acid in aqueous ammonia, each of these reactors being independently maintained at a constant and controlled temperature T1, T2, ... Tn respectively, the temperature of the array of reactors increasing between two consecutive reactors.

3. Process according to Claim 2, in which the temperature T1 of the reactor R1 is from 15 to 25°C and the temperature Tn of the final reactor Rn is from 26 to 40°C.

4. Process according to either one of Claims 2 and 3, in which the control of the temperature of each reactor is carried out by combining the control of the inlet temperature of the ingredients 11-bromoundecanoic acid and aqueous ammonia during stage i), the control of the degassing of the excess ammonia in each reactor of stage ii) and the use of thermoregulated systems for heating the reactors R1 to Rn.

5. Process according to any one of Claims 2 to 4, in which, the reaction medium being transferred from a given reactor to the consecutive reactor and the mean residence time in each reactor being from 1 h to 30 h, the total duration of the reaction which makes possible the complete consumption of 11-bromoundecanoic acid is from 20 to 80 h.

6. Process according to Claim 1, in which stage ii) takes place in an array of reactors in parallel R'1, R'2 ... R'n, 2 ≤ n ≤ 25, each of these reactors being subjected to a variable temperature.

7. Process according to Claim 6, in which the starting temperature of each reactor is from 15 to 25°C and the final temperature of each reactor is from 26 to 40°C.

8. Process according to either one of Claims 6 and 7, in which each reactor operates according to loading/reaction/emptying cycles with an offset in time equivalent to the reaction time divided by the number of reactors n, in order to maintain constant, regular and continuous feeding and withdrawing at the inlet and outlet of the array of reactors.

9. Process according to Claim 1, in which stage ii) takes place non-continuously in a reactor subjected to a variable temperature Tf, said reactor having a program for rise in temperature over a given number of stationary phases or a gradient for rise in temperature, the duration of which depends on the temperature chosen.

10. Process according to Claim 9, in which the starting temperature of the reactor is from 15 to 25°C and the final temperature of the reactor is from 26 to 40°C.

11. Process according to one of Claims 6, 7, 9 and 10, in which the duration of the reaction, which makes possible the complete consumption of 11-bromoundecanoic acid, varies from 20 h to 80 h according to the starting and final temperatures and the temperature rise program.

12. Process according to one of Claims 9 to 11, in which a rise in temperature from 22°C to 32°C is imposed on the reaction medium while observing 6 stationary phases of 12 h 30 min at 22°C, 24°C, 26°C, 28°C, 30°C and 32°C.

13. Process according to any one of the preceding claims, additionally comprising a stage iii) of filtration, washing and pulling dry, carried out on the 11-aminoundecanoic acid obtained at the end of stage ii).

14. Process according to Claim 13, additionally comprising a stage iv) of purification of the 11-aminoundecanoic acid obtained at the end of stage iii), preferably by the following sequence of operations: redissolution, filtration, crystallization, filtration, washing and pulling dry.

15. Process according to Claim 14, additionally comprising a stage v) of recovery of the residual 11-aminoundecanoic acid in the various filtrates and wash liquors obtained in stages iii) and/or iv), by degassing of the ammonia, liquid/liquid extraction, crystallization, filtration and washing.

16. Process according to either of Claims 14 and 15, additionally comprising a final stage of drying the 11-aminoundecanoic acid obtained in stage iv), optionally with the addition of that obtained in stage v).

17. Process according to any one of Claims 1 to 12, in which the level of aminodiundecanoic acid in the crude reaction product obtained is less than 3500 ppm, preferably less than 2500 ppm.

18. Process according to any one of Claims 1 to 12 and 17, in which the duration of the ammonolysis stage resulting in the complete consumption of 11-bromoundecanoic acid is less than 75 h.
